# EUROPÄISCHE PATENTSCHRIFT

(11) **EP 2 241 544 B1**
(45) Veröffentlichungstag und Bekanntmachung des Hinweises auf die Patenterteilung: **04.07.2012**
(21) Anmeldenummer: 09014345.4
(22) Anmeldetag: 17.11.2009
(51) Int. Cl.: C07C 29/80, C07C 31/22, C11D 19/00, C07C 29/76

(54) **Verfahren zur kontinuierlichen Herstellung von Reinglycerin aus Kaliumsulfat enthaltendem Rohglycerin**
Process for the continuous preparation of pure glycerol from crude glycerol containing potassium sulfate
Procédé de préparation en continu de glycerol pur à partir d'un brut de glycerol contenant du sulfate de potassium

(30) Priorität: 15.04.2009 DE 102009017098
(43) Veröffentlichungstag der Anmeldung: 20.10.2010
(73) Patentinhaber: Lurgi GmbH, 60439 Frankfurt am Main (DE)
(72) Erfinder: Golbach, Marco, 60439 Frankfurt am Main (DE)

(56) Entgegenhaltungen:
- EP-A2- 0 168 893
- WO-A1-2009/006923
- US-A- 2 151 990
- US-A- 4 655 879

## Beschreibung

Die Erfindung betrifft ein Verfahren und eine Vorrichtung zur kontinuierlichen Herstellung von Reinglycerin aus Kaliumsulfat enthaltendem, aus der Umesterung nativer Fette oder Öle stammendem Rohglycerin. Unter dem Begriff MONG (Matter Organic Non Glycerol) werden alle im Rohglycerin vorhandenen und im Verlauf der Herstellung von Reinglycerin entstandenen organischen Verunreinigungen zusammengefasst.

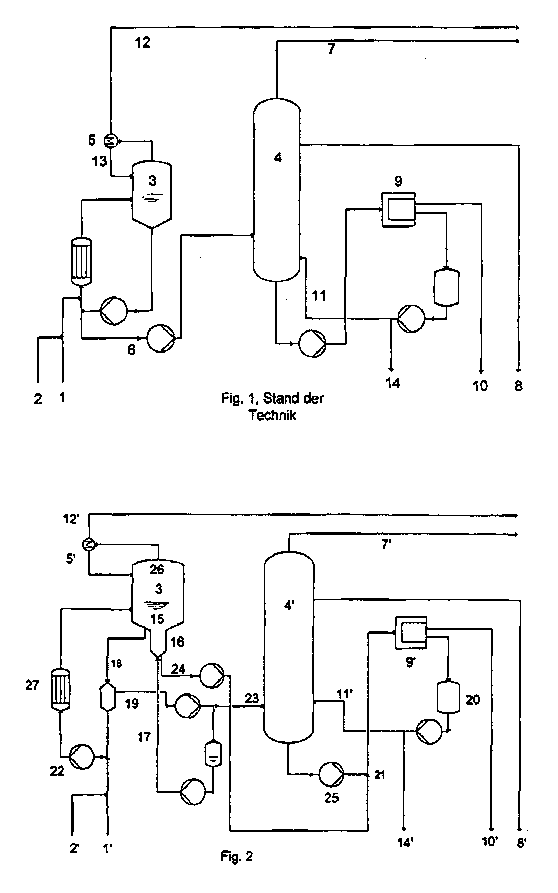

Das in Fig.1 als Grundfließbild dargestellte, zum Stand der Technik gehörende Verfahren, dessen Prinzip auch in der Broschüre "Biodiesel" der Firma Lurgi GmbH, Frankfurt/M, Stand Okt. 2008, dargestellt ist, hat sich in der Praxis für die Verarbeitung von mit Natriumchlorid beladenem Rohglycerin zu Reinglycerin bewährt. Bei diesem Verfahren wird das über Leitung (1) zugeführte Rohglycerin mit über Leitung (2) zugeführter Natronlauge zur Verseifung des MONG versetzt, der dadurch in der Rektifikationsanlage leichter aus dem Glycerin abtrennbar ist. Außerdem wird durch die Zugabe der Lauge zum Korrosionsschutz der Anlage der pH-Wert der Glycerinlösung in den basischen Bereich 7,5 bis 9,5, vorzugsweise 8 bis 9, verschoben.
Das so behandelte Rohglycerin wird in die Umlaufleitung eines Zwangsumlaufverdampfers gegeben. Hierin wird Wasser und Methanol oder Ethanol, zum größten Teil aus der Glycerinlösung ausgedampft.

Die Dämpfe werden über Leitung (12) von einer nicht dargestellten Vakuumanlage aus dem Brüdenabscheider (3) des Verdampfers abgezogen. In Kondensator (5) wird mitverdampftes Glycerin auskondensiert und fließt über Leitung (13) in den Brüdenabscheider zurück.
Im Zwangsumlaufverdampfer bleibt das Natriumchlorid in der Glycerinlösung gelöst und kristallisiert nicht aus. Aus dem Verdampferkreislauf wird über Leitung (6) die Glycerinlösung in eine Rektifikationskolonne gegeben, in der restliches Wasser und Methanol oder Ethanol als Kopfprodukt abgetrennt und über Leitung (7) von einer nicht dargestellten Vakuumanlage abgezogen werden. Das Glycerin verlässt die Rektifikationskolonne (4) als Seitenprodukt über Leitung (8) mit einer für Reinglycerin geforderten Konzentration von mindestens 99,5 Gew-% und wird zur Entfernung von Farb- und Geruchsstoffen in eine nicht dargestellte Adsorptionsanlage gegeben.
Das Sumpfprodukt der Rektifikationskolonne (4) besteht aus MONG, Glycerin und auskristallisiertem Natriumchlorid. Es wird über eine Vollmantelschneckenzentrifuge (9) im Kreis geführt. Das von der Zentrifuge abgetrennte Natriumchlorid wird über Leitung (10) aus dem Prozess ausgetragen. Das vom Natriumchlorid, bis auf einen Restgehalt befreite Sumpfprodukt wird zum größeren Teil über Leitung (11) in den Sumpf der Rektifikationskolonne (4) zurückgeführt und zum kleineren Teil über Leitung (14) aus dem Prozess ausgeschleust. Durch diese Kreislaufführung ist es möglich, ohne die Glycerinausbeute der Rektifikationskolonne zu verschlechtern, den MONG mit einem relativ hohen Glyceringehalt, und damit dünnflüssiger, als es ohne den Kreislauf möglich wäre, in die Zentrifuge (9) zu geben und dadurch deren Abscheideleistung zu erhöhen. Der aus dem Kreislauf abgezogene MONG kann, durch die mittels der Zentrifuge (9) erfolgte Absenkung des Salzgehalts, als Brennmaterial verwendet werden. Dies ist für die energetische Optimierung und somit die Wirtschaftlichkeit des Verfahrens von Bedeutung.

Ein ähnliches Verfahren ist in WO 2009/006923 A1 beschrieben. Dieses Verfahren unterscheidet sich von dem oben beschriebenen dadurch, dass das Glycerin zum größten Teil zusammen mit seinem Wasseranteil durch Verdampfen von dem salzhaltigen MONG abgetrennt wird, ohne dass eine gezielte Einstellung der Reinheit des abgetrennten Glycerins mit diesem Verfahren durchgeführt wird. Die Abtrennung des auskristallisierten Salze aus dem MONG erfolgt, wie im eingangs beschriebenen Verfahren, indem der MONG aus dem Sumpf einer Destillationsblase im Kreislauf über eine Zentrifuge gefahren wird.

US 4,655,879 dagegen beschreibt ein Verfahren zur destillativen Aufarbeitung von Glycerin bei dem die festen Verunreinigungen, wie Seifen und Salze, mittels eines Dünnschichtverdampfers vom Glycerin abgetrennt werden. Dabei wird das Rohglycerin, nach einer Vorbehandlung mit Lauge und Luftsauerstoff, mit allen verdampfbaren Bestandteilen, im Dünnschichtverdampfer von den festen Verunreinigungen abgedampft. Auf diese Weise können die nachfolgenden Reinigungsschritte des Glycerins, durch Rektifikation und Adsorption, unbeeinträchtigt von den festen Verunreinigungen erfolgen.

Ein Nachteil der Verfahren gemäß Fig. 1 und gemäß WO 2009/006923 A1 ist es, dass sie bei der Herstellung von Reinglycerin aus mit Kaliumsulfat beladenem Rohglycerin nicht in der Lage sind, die Salzbeladung des MONG soweit zu senken, dass er als Brennmaterial einsetzbar ist. Ursache dafür, dass Kaliumsulfatkristalle in einer Vollmantelschneckenzentrifuge schwieriger abzuscheiden sind als Natriumchloridkristalle, ist die Form und die Größe der Kaliumsulfatkristalle. Anders als die körnigen Natriumchloridkristalle, haben Kaliumsulfatkristalle eine Nadel- oder auch Dendrit-Form. Durch diese strauchartige Form benötigen sie eine größere Masse bzw. Kristallgröße als Natriumchloridkristalle, damit sie mittels Vollmantelschneckenzentrifuge aus dem organischen Rückstand abtrennbar sind. Diese Verfahren nach dem Stand der Technik bieten aber keine Möglichkeit, die Größe der Kristalle zu beeinflussen.

Nachteilig am Verfahren gemäß US 4,655,879 sind die hohen Investitionskosten die ein Dünnschichtverdampfer erfordert.

Aufgabe der Erfindung ist es daher, ein Verfahren bereitzustellen, dass auch bei der Verwendung von mit Kaliumsulfat beladenem Rohglycerin der MONG soweit vom anorganischen Salz befreit werden kann, dass er als Brennmaterial einsetzbar ist.

Die Aufgabe wird durch das erfindungsgemäße Verfahren gelöst, bei dem durch die Zugabe von Lauge der MONG verseift und ein pH-Wert im Bereich von 7,5 bis 9,5, bevorzugt im Bereich 8 bis 9, eingestellt wird, anschließend in einem Zwangsumlaufverdampfer Wasser und Methanol- oder Ethanol ausgedampft werden und Kaliumsulfat auskristallisiert wird, wobei die gewünschte durchschnittliche Kristallgröße im Zwangsumlaufverdampfer durch Einstellung der Dichte der umlaufenden Suspension eingestellt wird, die glycerinreiche Phase über einen in der Umlaufleitung des Verdampfers installierten Abscheider, unter Zurückhaltung der Kaliumsulfatkristalle, aus dem Verdampfer abgezogen wird und in einer Rektifikationskolonne zumindest auf 99,5 Gew.-% aufkonzentriert und anschließend durch Adsorption von Farb- und Geruchsstoffen befreit wird, das Sumpfprodukt der Rektifikationskolonne und die aus dem Zwangsumlaufverdampfer abgezogene, aus Glycerin und Kaliumsulfat bestehende Trübe, zusammen in einer Vollmantelschneckenzentrifuge von den Kaliumsulfatkristallen befreit werden und der aus der Vollmantelschneckenzentrifuge abgezogene, im wesentlichen aus MONG bestehende, organische Rückstand weniger als 4 Gew-%, bevorzugt weniger als 3 Gew-%, Kaliumsulfat enthält.

Zur Einstellung der durchschnittlichen Kristallgröße im Zwangsumlaufverdampfer wird die dazu proportionale Dichte der umlaufenden Suspension aus glycerinreicher Lösung und Kaliumsulfatkristallen gemessen. Diese Messung erfolgt kontinuierlich in der Umlaufleitung. Die Regelung der Suspensionsdichte erfolgt über die Einstellung der aus dem Verdampferbehälter, durch das Austragsrohr, pro Zeiteinheit, in Form einer Salztrübe, abgezogenen Menge an Kaliumsulfatkristallen.
In einer bevorzugten Ausgestaltung der Erfindung wird durch die Einspeisung kristallfreier Lösung im unteren Teil des Austragsrohrs eine aufwärts gerichtete Strömung erzeugt, indem deren Volumenstrom größer als der Volumenstrom der abgezogenen Salztrübe eingestellt wird. Durch die Einstellung der Stärke der aufwärtsgerichteten Strömung wird das Absinken von Kristallen im Austragsrohr unterhalb einer gewählten Größe verhindert.
Auch der Umfang der sekundären Keimbildung soll durch eine schonende Behandlung der bereits gebildeten Kristalle und einen möglichst niedrigen Eintrag mechanischer Energie gering gehalten werden, um das Kristallwachstum zu fördem. Aus diesem Grund wird für den Umlauf der Suspension eine Rohrbogenpropellerpumpe eingesetzt.

Die glycerinreiche Lösung wird über einen in der Umlaufleitung installierten Abscheider, unter weitgehender Zurückhaltung der Kaliumsulfatkristalle, abgezogen.
Ein Teilstrom dieser glycerinreichen Lösung wird zur Einstellung der für die weitere Verarbeitung geeigneten Dichte der Salztrübe, in das Austragsrohr eingespeist.
Die über den Abscheider aus dem Verdampferkreislauf abgezogene glycerinreiche Phase wird zur Einstellung der für Reinglycerin geforderten Konzentration in eine Rektifikationskolonne eingespeist. Dort wird restliches Wasser als Kopfprodukt abgetrennt und MONG als Sumpfprodukt. Um den MONG dünnflüssig zur Abtrennung des Salzes in die Zentrifuge geben zu können, wird er zwischen Ko-Ionnensumpf und Zentrifuge im Kreislauf gefahren. Aus diesem Kreislauf wird, hinter der Zentrifuge, ein Teilstrom als fertiges, vom Salz befreites und zur Verbrennung geeignetes Abfallprodukt, aus dem System ausgeschleust.
Um den Glyceringehalt des MONG, und damit seine Viskosität, unabhängig von der Betriebsweise der Rektifikationskolonne einstellen zu können, kann dem Kolonnensumpf stromwärts eine Nachdestillationsblase nachgeschaltet sein.

Das erfindungsgemäße Verfahren wird nachfolgend, an Hand eines Ausführungsbeispiels in Verbindung mit den Zeichnungen erläutert.

Es zeigen:
Fig. 1 Verfahren gemäß Stand der Technik,
Fig. 2 schematisch eine Vorrichtung zur kontinuierlichen Herstellung von Reinglycerin aus Kaliumsulfat enthaltendem Rohglycerin gemäß einer bevorzugten Ausführungsform der Erfindung,
Fig. 3 eine bevorzugte Ausgestaltung des erfindungsgemäßen Abscheiders.

Bei dem erfindungsgemäßen Verfahren wird das aus einem Tanklager kommende Rohglycerin (1') mit 25%-iger Natronlauge (2') versetzt, mittels eines in Fig.2 nicht dargestellten Wärmetauschers im Gegenstrom vom die Rektifikationskolonne (4') über Leitung (8') verlassenden Glycerin auf 95°C aufgeheizt und in die Umlaufleitung des Zwangsumlaufverdampfers eingespeist. Die umlaufende Glycerinlösung wird von einer Rohrbogenpropellerpumpe (22), mit einem Laufraddurchmesser von 300 mm, gefördert. Durch den Umlauferhitzer (27) wird die Lösung auf 120°C erhitzt.
Durch die Absaugung der Dämpfe Ober Leitung (12') wird im Dampfraum (26) den Druck auf 55 mbar, abs gesenkt. Der Wassergehalt des Rohglycerins wird im Verdampfer von 10% auf 3% gesenkt.
Durch das Abdampfen des Wassers stellt sich im Verdampferbehälter (3) ein metastabiler Übersättigungszustand ein, es kommt zum Auskristallisieren des Kaliumsulfats indem verhandene Kristalle vergrößert werden und durch sekundäre Keimbildung. Die Kristalle haben eine höhere Dichte als die Lösung und sinken daher in den Bodenbereich des Verdampferbehälters (15) und von dort weiter in das Austragsrohr (16) ab. Durch Einspeisung des Stroms 17 wird die Dichte der im Austragsrohr gebildeten Salztrübe eingestellt. Es wird ein mittlerer Sinkgeschwindigkeitsäquivalentdurchmesser von 0,1 mm für die Kaliumsulfatkristalle angestrebt.
Der Sinkgeschwindigkeitsäquivalentdurchmesser wird unregelmäßig geformten Kristallen, zu denen Kaliumsulfatkristalle gehören, zugeordnet, die bei gleicher Dichte, die gleiche Sinkgeschwindigkeit haben wie kugelförmige Kristalle mit diesem Durchmesser. Diese Methode zur Beschreibung von Kristallgrößen ist beschrieben in Leschonski et al.: Teilchengrößenanalyse, Artikelserie in Chemie-Ingenieur-Technik 46 (1974) und 47 (1975).

Der Verdampferbehälter hat einen Durchmesser von 2,8 m und eine Gesamthöhe (ohne Austragsrohr) von 9 m. Das Austragsrohr hat eine Länge von 4 m und einen Durchmesser von 0,3 m. Das Flüssigkeitsvolumen im Verdampferbehälter beträgt 23,6 m³ (inclusive Austragsrohr).

Die aus dem Verdampfer ausgetragene, aus Glycerin und Kaliumsulfatkristallen bestehende Trübe wird über Leitung (21) in den Kreislauf des Sumpfproduktes der Rektifikationskolonne, der über die Zentrifuge führt, eingespeist.
Die Glycerinlösung wird über den Dekanter (19) aus dem Verdampferkreislauf abgezogen und Ober Rohrleitung (23) in die Rektifikationskolonne gegeben.

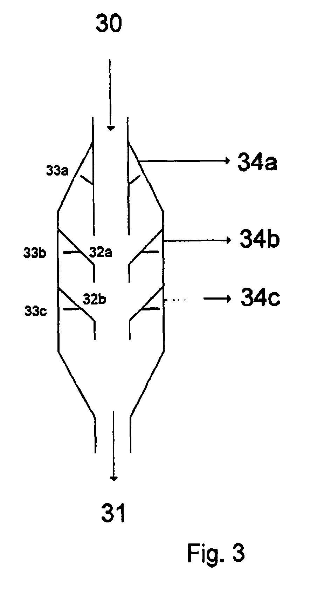

Gemäß des in Fig. 3 der Zeichnung dargestellten Längsschnitts ist der Abscheider als Dekanter ausgestaltet und besteht aus einem zylindrischen Behälter mit einem konischen Deckel und einem konischen Boden. Der Abscheider wird in senkrechter Stellung in die Umlaufleitung, stromwärts nach dem Verdampferbehälter, integriert. Die Suspension aus Glycerinlösung und Kaliumsulfatkristallen durchströmt ihn von oben (30) nach unten (31). Die Zulaufleitung taucht bis in den zylindrischen Teil des Behälters ein. Im zylindrischen Teil sind untereinander zwei Trichter 32 a und b installiert, deren Auslauf den gleichen Durchmesser wie die Zulaufleitung hat. Die Trichter sind mit dem oberen Rand umlaufend mit der Innenseite des Behälters verbundenen.
Auf der Außenseite der in den Behälter eintauchenden Zulaufleitung und auf der Außenseite der Trichter ist jeweils im Bereich von 30 bis 70% der Seitenhöhe ein umlaufendes Trennblech 33 a, b, c befestigt, dessen freie Kante einen umlaufenden Spalt mit der Innenseite des Behälters bildet. Im Bereich oberhalb des Trennblechs befindet sich jeweils ein Rohrleitungsanschluss zum Abziehen der an dieser Stelle weitgehend von Kaliumsulfatkristallen befreiten glycerinreichen Phase. Die drei Abzugleitungen 34 a, b und c werden zu einer, in Fig.3 nicht dargestellten, einzigen Abzugsleitung zusammengefasst.

In diesem Beispiel hat der Dekanter (19) eine Gesamthöhe von 3,4 m und einen Durchmesser von 1,2 m. Der umlaufende Spalt zwischen der Innenseite der Behälterwand und den Trennblechen 33 a, b und c ist zwischen 1,5 und 4,5 mm, vorzugsweise 3 mm weit.

Die Rektifikationskolonne wird mit einer Temperatur von 170°C im Kolonnensumpf betrieben. Wasser und Methanol oder Ethanol, werden als Kopfprodukt abgetrennt und über Leitung (7') von einem, nicht dargestellten, Vakuumsystem abgezogen.

Das gereinigte Glycerin verlässt die Kolonne als Seitenprodukt über Leitung (8') mit einem Glyceringehalt von mindestens oder gleich 99,5 Gew-%. Es wird anschließend einer, in Fig.2 nicht dagestellten, Adsorptionsbehandlung, z.B. mit Aktivkohle, zur Entfernung von Farb- und Geruchsstoffen unterzogen. Das so behandelte Reinglycerin kann dann als Pharmaglycerin verwendet werden.
Das im Rohglycerin enthaltende Kaliumsulfat kristallisiert zu ca. zwei Dritteln im Verdampfer und zu ca. einem Drittel im Sumpf der Rektifikationskolonne aus.

Das Sumpfprodukt der Rektifikationskolonne wird über die Pumpe (25), die Vollmantelschneckezentrifuge (9') und den Zwischenbehälter (20) im Kreis geführt. Aus diesem Kreislauf wird ein Teilstrom als beinahe salzfreier, als Brennmaterial geeigneter MONG aus dem Prozess über Leitung (14') ausgeschleust. Das von der Zentrifuge aus dem Sumpfprodukt abgetrennte Salz wird aus dem Verfahren, Ober Leitung (10'), ausgetragen.

Mit dem erfindungsgemäßen Verfahren wird somit ein energetisch vorteilhaftes, rückstandsarmes Verfahren mit verbesserter Wirtschaftlichkeit vorgeschlagen.

### Stoffstromtabelle

| Stoffstrom | | 1' | 2' | 12' | 23 | 17 | 24 | 18 | 7' | 25 | 9' | 14' | 11' | 8' |
|---|---|---|---|---|---|---|---|---|---|---|---|---|---|---|
| Glycerin | Gew-% | 83,5 | | 0,5 | 92,7 | 94.5 | 67 | 81 | 1 | 40 | 6 | 47 | 47 | 99,8 |
| Wasser | Gew-% | 10 | | 94,5 | 3 | 1 | 0,7 | 0,9 | 97 | 0 | 0 | 0 | 0 | 0,2 |
| K₂SO₄ | Gew-% | 3 | | 0 | 1 | 1 | 30 | 15 | 0 | 3 | 91 | 1 | 1 | 0 |
| MONG | Gew-% | 3 | | 0 | 3,3 | 3,4 | 2,4 | 3 | 0 | 57 | 3 | 52 | 52 | 0,1 |
| Methanol | Gew-% | 0,5 | | 5 | 0 | 0 | 0 | 0 | 2 | 0 | 0 | 0 | 0 | 0 |
| Dichte | kg/m³ | 1,200 | 1,270 | | 1,192 | 1,196 | 1,633 | 1.191 | | | 2,600 | 1,200 | 1,200 | 1,165 |
| Temperatur | °C | 45 | 30 | 80 | 120 | 120 | 120 | 120 | 43 | 175 | | | 175 | 158 |
| Druck | bar | 5 | 3 | 0,050 | | | 3 | 2 | 0,003 | 3 | 1,0 | 1,0 | 2,0 | |
| Durchfluss | kg/h | 23.807 | 40 | 2.300 | 20.197 | 600 | 1.721 | 948 | 570 | 9.150 | 773 | 1.207 | 8.850 | 18.750 |

## Patentansprüche

1. Verfahren zur kontinuierlichen Herstellung von Reinglycerin aus Kaliumsulfat enthaltendem, aus der Umesterung von Fette oder fetten Ölen stammendem Rohglycerin, das aus zumindest 80 Gew-% Glycerin besteht und 5 bis 15 Gew-% Wasser, 0,5 bis 5 Gew-% Kaliumsulfat, 0,5 bis 5 Gew-% MONG und 0,2 bis 2 Gew-% Methanol oder Ethanol enthält, bei dem durch die Zugabe von Lauge der MONG verseift und ein pH-Wert im Bereich von 7,5 bis 9,5, bevorzugt im Bereich 8 bis 9, eingestellt wird, anschließend in einem Zwangsumlaufverdampfer Wasser und Methanol oder Ethanol ausgedampft werden und Kaliumsulfat auskristallisiert wird, wobei die gewünschte durchschnittliche Kristallgröße im Zwangsumlaufverdampfer durch Einstellung der aus dem Verdampferbehälter, durch das Austragsrohr, pro Zeiteinheit, in Form einer Salztrübe, abgezogenen Menge an Kaliumsulfatkristallen eingestellt wird, die glycerinreiche Phase über einen in der Umlaufleitung des Verdampfers installierten Abscheider, unter Zurückhaltung der Kaliumsulfatkristalle, aus dem Verdampfer abgezogen wird und in einer Rektifikationskolonne zumindest auf 99,5 Gew.-% aufkonzentriert und anschließend durch Adsorption von Farb- und Geruchsstoffen befreit wird, da s Sumpfprodukt der Rektifikationskolonne und die aus dem Zwangsumlaufverdampfer abgezogene, aus Glycerin und Kaliumsulfat bestehende Trübe zusammen in einer Vollmantelschneckenzentrifuge von den Kaliumsulfatkristallen befreit werden und der aus der Vollmantelschneckenzentrifuge abgezogene, im wesentlichen aus MONG bestehende, organische Rückstand weniger als 4 Gew-%, bevorzugt weniger als 3 Gew-%, Kaliumsulfat enthält.

2. Verfahren nach Anspruch 1, **dadurch gekennzeichnet, dass** die Dichte der im Zwangsumlaufverdampfer umlaufenden Suspension in der Umlaufleitung zwischen Verdampferbehälter und Abscheider (19) gemessen und durch Einstellung der am Boden des Verdampferbehälters pro Zeiteinheit abgezogenen Menge an Salztrübe geregelt wird.

3. Verfahren nach einem der vorhergehenden Ansprüche, **dadurch gekennzeichnet, dass** die Satztrübe durch ein am Boden des Zwangsumlaufverdampfers angebrachtes Austragsrohr (16) ausgetragen wird, wobei durch Einspeisen von kristallfreier Lösung in das Austragsrohr, die Dichte der ausgetragenen Salztrübe eingestellt wird.

4. Verfahren nach Anspruch 3, **dadurch gekennzeichnet, dass** die kristallfreie Lösung im unteren Teil des Austragsrohrs (16) eingespeist wird und deren Volumenstrom um so viel größer als der Volumenstrom der abgezogenen Salztrübe eingestellt wird, dass durch die daraus resultierende, aufwärtsgerichtete Strömung das Absinken von Kristallen unterhalb einer gewählten Größe im Austragsrohr verhindert wird.

5. Verfahren nach einem der vorhergehenden Ansprüche, **dadurch gekennzeichnet, dass** zum Abtrennen der zur weiteren Behandlung in der Rektifikationskolonne vorgesehene Anteil der Lösung als Abscheider (19) ein Dekanter verwendet wird, der in senkrechter Stellung in die Umlaufleitung eingebaut ist und von der Suspension von oben nach unten durchströmt wird, der aus einem zylindrischen Behälter mit einem konischen Deckel und einem konischen Boden besteht, bei dem die Zulaufleitung bis in den zylindrischen Teil des Behälters eintaucht, bei dem im zylindrischen Teil mindestens ein Trichter installiert ist, dessen Auslauf den gleichen Durchmesser wie die Zulaufleitung hat und der mit dem oberen Rand umlaufend mit der Innenseite des Behälters verbunden ist, bei dem auf der Außenseite der in den Behälter eintauchenden Zulaufleitung und auf der Außenseite des Trichters jeweils im Bereich von 30 bis 70% der Seitenhöhe ein umlaufendes Trennblech befestigt ist, dessen freie Kante einen umlaufenden Spalt mit der Innenseite des Behälters bildet, wobei sich im Bereich oberhalb des Trennblechs jeweils ein Rohrleitungsanschluss zum Abziehen der an dieser Stelle weitgehend von Kaliumsulfatkristallen befreiten glycerinreichen Phase befindet.

6. Verfahren nach einem der vorhergehenden Ansprüche, **dadurch gekennzeichnet, dass** das gewonnene Reinglycerin nach adsorptive Nachbehandlung als Pharmaglycerin verwendet wird.

## Claims

1. A process for the continuous preparation of pure glycerol from crude glycerol containing potassium sulfate and originating from the transesterification of fats or fatty oils, which consists of at least 80 wt-% glycerol and contains 5 to 15 wt-% water, 0.5 to 5 wt-% potassium sulfate, 0.5 to 5 wt-% MONG and 0.2 to 2 wt-% methanol or ethanol, in which the MONG is saponified by adding lye and a pH-value in the range from 7.5 to 9.5, preferably in the range from 8 to 9, is adjusted, subsequently water and methanol or ethanol are evaporated in a forced-circulation evaporator and potassium sulfate is crystallized out, wherein the desired average crystal size in the forced-circulation evaporator is adjusted by adjusting the amount of potassium sulfate crystals withdrawn, per unit time, from the evaporator tank through the discharge tube in the form of a salt slurry, the phase rich in glycerol is withdrawn from the evaporator via a separator installed in the circulation conduit of the evaporator, by retaining the potassium sulfate crystals, and in a rectification column is concentrated up to at least 99.5 wt-% and subsequently liberated from dyes and odorants by adsorption, the bottom product of the rectification column and the slurry withdrawn from the forced-circulation evaporator and consisting of glycerol and potassium sulfate together are liberated from the potassium sulfate crystals in a solid-bowl scroll-type centrifuge and the organic residue withdrawn from the solid-bowl scroll-type centrifuge, which substantially consists of MONG, contains less than 4 wt-%, preferably less than 3 wt-% potassium sulfate.

2. The process according to claim 1, **characterized in that** the density of the suspension circulating in the forced-circulation evaporator is measured in the circulation conduit between evaporator tank and separator (19) and is controlled by adjusting the amount of salt slurry withdrawn at the bottom of the evaporator tank per unit time.

3. The process according to any of the preceding claims, **characterized in that** the salt slurry is discharged through a discharge tube (16) mounted at the bottom of the forced-circulation evaporator, wherein by feeding crystal-free solution into the discharge tube the density of the salt slurry discharged is adjusted.

4. The process according to claim 3, **characterized in that** the crystal-free solution is fed into the lower part of the discharge tube (16) and its volumetric flow rate is so much greater than the volumetric flow rate of the salt slurry withdrawn, that the resulting upwardly directed flow prevents the settling of crystals below a chosen size in the discharge tube.

5. The process according to any of the preceding claims, **characterized in that** for separating the part of the solution provided for the further treatment in the rectification column a decanter is used as separator (19), which is incorporated in the circulation conduit in a vertical position and is traversed by the suspension from top to bottom, which consists of a cylindrical tank with a conical lid and a conical bottom, in which the supply conduit extends down into the cylindrical part of the tank, in which in the cylindrical part at least one funnel is installed, whose outlet has the same diameter as the supply conduit and whose upper edge is circumferentially connected with the inside of the tank, in which on the outside of the supply conduit immersed into the tank and on the outside of the funnel a circumferential divider is attached each in the range of 30 to 70% of the side depth, whose free edge forms a circumferential gap with the inside of the tank, wherein in the region above the divider there is each located a conduit connection for withdrawing the phase rich in glycerol, which at this point is largely liberated from potassium sulfate crystals.

6. The process according to any of the preceding claims, **characterized in that** the pure glycerol obtained is used as pharmaceutical glycerol after an adsorptive aftertreatment.

## Revendications

1. Procédé de préparation en continu de glycérol pur à partir de glycérol brut contenant du sulfate de calcium provenant de la transestérification de graisses ou d'huiles grasses, constitué d'au moins 80% en poids de glycérol et contenant de 5 à 15% en poids d'eau, de 0,5 à 5% en poids de sulfate de potassium, de 0,5 à 5% en poids de MONG et de 0,2 à 2% en poids de méthanol ou d'éthanol, dans lequel on saponifie la MONG par l'addition de lessive et on établit un pH dans la plage de 7,5 à 9,5, de préférence dans la plage de 8 à 9, ensuite on évapore de l'eau et du méthanol ou de l'éthanol dans un évaporateur à circulation forcée et on sépare du sulfate de calcium par cristallisation, la dimension moyenne souhaitée des cristaux dans l'évaporateur à circulation forcée étant réglée en réglant la quantité de cristaux de sulfate de calcium retirée par unité de temps sous la forme d'une boue de sel de la cuve de l'évaporateur par le tuyau d'évacuation, on retire de l'évaporateur, tout en retenant les cristaux de sulfate de calcium, la phase riche en glycérol par un séparateur monté dans le conduit de recirculation de l'évaporateur et on la concentre à au moins 99,5% en poids dans une colonne de rectification et ensuite on la débarrasse en adsorbant des substances colorantes et odoriférantes, on débarrasse le produit de queue de la colonne de rectification et la boue, retirée de l'évaporateur à circulation forcée et constituée de glycérol et de sulfate de calcium, ensemble dans une centrifugeuse à vis à bol plein, des cristaux de sulfate de calcium et le résidu organique, soutiré de la centrifugeuse à vis à bol plein et constitué essentiellement de MONG, contient moins de 4% en poids, de préférence moins de 3% en poids, de sulfate de calcium.

2. Procédé suivant la revendication 1, **caractérisé en ce que** l'on règle la masse volumique de la suspension circulant dans l'évaporateur à circulation forcée, telle que mesurée dans le conduit de recirculation entre la cuve de l'évaporateur et le séparateur (19), en réglant la quantité de boue de sel soutirée par unité de temps au fond de la cuve de l'évaporateur.

3. Procédé suivant l'une des revendications précédentes, **caractérisé en ce que** l'on soutire la boue de sel par un tuyau (16) d'évacuation mis sur le fond de l'évaporateur à circulation forcée, la masse volumique de la boue de sel soutirée étant réglée par injection de solution exempte de cristaux dans le tuyau d'évacuation.

4. Procédé suivant la revendication 3, **caractérisé en ce que** l'on injecte la solution exempte de cristaux dans la partie inférieure du tuyau (16) d'évacuation et on en règle le courant en volume pour qu'il soit plus grand que le courant en volume de la boue de sel soutirée, de manière à empêcher par le courant ascendant qui s'ensuit la descente de cristaux inférieurs à une dimension sélectionnée dans le tuyau d'évacuation.

5. Procédé suivant l'une des revendications précédentes, **caractérisé en ce que**, pour la séparation de la proportion de la solution prévue pour le traitement supplémentaire dans la colonne de rectification, on utilise comme séparateur (19) une décanteuse, qui est montée en position verticale dans le conduit de recirculation et qui est parcourue de haut en bas par la suspension, décanteuse qui est constituée d'une cuve cylindrique ayant un couvercle conique, le conduit d'amenée pénétrant jusque dans la partie cylindrique de la cuve, au moins un entonnoir étant monté dans la partie cylindrique, entonnoir dont la sortie a le même diamètre que le conduit d'amenée et qui, par le bord supérieur faisant le tour, communique avec le côté intérieur de la cuve, dans lequel, du côté extérieur du conduit d'amenée plongeant dans la cuve et du côté extérieur de l'entonnoir, est fixée, respectivement dans la plage de 30 à 70% de la hauteur du côté, une tôle de séparation faisant le tour, dont le bord libre forme un intervalle faisant le tour avec le côté inférieur de la cuve, un raccord de conduite tubulaire pour le retrait de la phase riche en glycérol débarrassée dans une grande mesure en ce point de cristaux de sulfate de calcium se trouvant respectivement dans la partie au-dessus de la tôle de séparation.

6. Procédé suivant l'une des revendications précédentes, **caractérisé en ce que** l'on utilise le glycérol obtenu, après un traitement ultérieur par adsorption, comme glycérol pharmaceutique.
